# DEMANDE DE BREVET EUROPEEN

(11) **EP 3 366 279 A1**
(43) Date de publication de la demande: **29.08.2018**
(21) Numéro de dépôt: 18156510.2
(22) Date de dépôt: 13.02.2018
(51) Int. Cl.: A61K 9/06, A61K 47/02, A61K 47/10, A61K 47/12, A61K 47/14, A61K 47/18, A61K 47/20, A61K 47/26, A61K 47/32, A61K 47/34, A61K 47/36, A61K 47/44, A61K 31/19, A01P 1/00, A61P 31/02

(54) **COMPOSITION DÉSINFECTANTE ET FILMOGÈNE DESTINÉE A ÊTRE APPLIQUÉE APRÈS LA TRAITE SUR UN TRAYON, CONTENANT DE L'ACIDE LACTIQUE**

(30) Priorité: 23.02.2017 FR 1751438; 13.04.2017 FR 1753265
(71) Demandeur: Hydrachim, 35370 Le Pertre (FR)
(72) Inventeur: FRETIN, Benoit, 35370 LE PERTRE (FR); GABLIN, Sandra, 53320 SAINT CYR LE GRAVELAIS (FR); LEBRETON, Rodrigue, 53320 SAINT CYR LE GRAVELAIS (FR)
(74) Mandataire: Ermeneux, Bertrand

(57) **Abrégé**

L'invention concerne une composition désinfectante et filmogène destinée à être appliquée après une traite sur le trayon d'un bovin, d'un ovin ou d'un caprin comprenant en milieu aqueux un composant filmogène et au moins une substance active désinfectante.

Selon l'invention, un telle composition comprend 5,4 à 11% en masse d'acide lactique à titre de substance active désinfectante et 1,955 à 4,45% en masse d'un agent tensio-actif non ionique à effet détergent et/ou mouillant seul ou en mélange, choisi parmi les alkyle polyglucosides, le PEG-75 lanoline, le polyoxyethylene-20-sorbitan monolaurate et le polymère d'oxyde de propylène et d'oxyde d'éthylène, un tampon de pH et un agent régulateur de pH, tel que de l'hydroxyde de sodium, pour ajuster le pH de la composition entre 2 et 4.

## Description

### 1. Domaine de l'invention

Le domaine de l'invention est celui de l'hygiène de la traite.

Plus précisément, l'invention concerne une composition désinfectante et filmogène destinée à être appliquée après la traite sur les trayons d'un bovin, d'un ovin ou d'un caprin.

### 2. Etat de la technique

Lors de la traite, une attention particulière doit être apportée à la désinfection des trayons et des soins cutanés doivent être administrés. Ceci permet de garantir la qualité du lait et de préserver la santé de l'animal.

Ces traitements consistent essentiellement à appliquer sur les trayons une solution liquide désinfectante, adoucissante et émolliente, par trempage ou par pulvérisation. L'application de cette solution vise à éliminer les germes, et notamment les germes d'*Escherichia Coli,* de *Streptococcus uberis* ou de *Staphylococcus aureus* présents à la surface de la peau des trayons. Elle permet également de soulager les irritations provoquées par la machine de traite et de soigner les éventuelles crevasses et gerçures sur la peau du trayon, qui peuvent entraîner des mammites en cas d'infection bactérienne.

Dans les élevages bovins, ovins ou caprins, il est connu d'appliquer, un produit désinfectant et filmogène sur les trayons après la traite, destiné à protéger, jusqu'à la prochaine traite, la peau et le sphincter des trayons des contaminations bactériennes pouvant survenir, par exemple, au contact d'une litière souillée,.

On connait des formulations désinfectantes et filmogènes s'appliquant après la traite, dont la substance active bactéricide est un iodophore. Des formulations de produits désinfectants et filmogènes contenant un iodophore sont décrits par exemple dans le document EP-B1-0 915 618. Les propriétés bactéricides de l'iode confèrent à ces formulations une efficacité convenable pour désinfecter les trayons.

Un inconvénient des iodophores est cependant leur manque de stabilité dans le temps.

Un autre inconvénient des iodophores est qu'ils peuvent être assimilés par voie systémique et qu'on peut ainsi retrouver des concentrations élevées d'iode dans le lait de l'animal.

Encore un inconvénient des iodophores est qu'ils doivent être stockés à des températures inférieures à 40°C, au risque de devenir instables.

On a également pensé à utiliser de la chlorexhidine, des sulfamides, ou du chlorite de sodium comme substance active bactéricide ou limitant le développement des souches bactériennes les plus courantes.

Un inconvénient de ces substances chimiques actives est qu'elles peuvent être irritantes et provoquer des effets secondaires, tels qu'une intolérance tissulaire. En outre, du fait de l'accoutumance à terme des germes pathogènes à certains de ces produits, il est nécessaire d'augmenter progressivement les doses utilisées, qui peuvent alors se révéler nocives, voire toxiques, pour l'animal.

Un autre inconvénient de ces substances actives, tout comme des iodophores, est qu'elles sont issues de l'industrie chimique, et ne sont pas d'origine végétale.

Afin de remédier à ces inconvénients, on a proposé d'utiliser des substances actives bactéricides extraites de végétaux, telles que l'extrait liquide de citrus.

Un inconvénient de l'extrait de citrus est qu'il n'est pas approuvé en tant que substance biocide par le règlement CE 526/2012.

### 3. Objectifs de l'invention

L'invention a donc notamment pour objectif de pallier les inconvénients de l'état de la technique cités ci-dessus.

Plus précisément l'invention a pour objectif de fournir une composition désinfectante et filmogène destinée à être appliquée après la traite, à partir de composés extraits de produits naturels.

Notamment, dans un mode de réalisation particulier de l'invention, l'invention a pour objectif de proposer une composition désinfectante et filmogène qui puisse être utilisée en agriculture biologique.

Un objectif de l'invention est également de fournir une telle composition qui inactive les micro-organismes pathogènes, tels que les bactéries et les levures, et notamment les germes d'*Escherichia Coli,* de *Streptococcus uberis* ou de *Staphylococcus aureus* et l'espèce de levure *Candida albicans.*

Notamment, l'invention a pour objectif, dans un mode de réalisation particulier de l'invention, de fournir une composition désinfectante dont l'activité bactéricide est conforme à la norme EN 1656 pour les souches bactériennes précitées et à la norme EN1657 pour les levures, et en particulier qui soit efficace à l'état pur en moins de 5 minutes sur ces souches, à 30°C et dans des conditions de désinfection des trayons.

L'invention a par ailleurs pour objectif de fournir une composition désinfectante et filmogène qui soit efficace sur l'intervalle de temps entre deux traites, soit entre 8 et 12 heures.

Un autre objectif de l'invention est de fournir une composition désinfectante et filmogène destinée à être appliquée après la traite qui ne provoque pas de réactions d'intolérance et d'irritations.

Encore un objectif de l'invention est de fournir une composition qui forme un film régulièrement réparti et d'épaisseur homogène sur le trayon.

L'invention a également pour objectif de proposer une composition désinfectante et filmogène qui puisse être retirée facilement du trayon par un simple lavage et essuyage humide avant la traite suivante, sans risque d'abrasion ou d'irritation du trayon.

Un objectif de l'invention est également de proposer une composition désinfectante et filmogène destinée à être appliquée après la traite qui soit d'un coût de revient réduit.

Encore un objectif de l'invention est de fournir une telle composition qui soit stable dans le temps et à des températures comprises entre 0°C et 30°C.

L'invention a également pour objectif de fournir une composition désinfectante et filmogène après-traite qui soit non toxique et non allergène pour l'animal ou pour l'éleveur.

### 4. Exposé de l'invention

La Demanderesse a mis en évidence, de façon inattendue, en cherchant à formuler une composition désinfectante et filmogène destinée à être appliquée après une traite sur le trayon d'un bovin, d'un ovin ou d'un caprin comprenant en milieu aqueux un composant filmogène et au moins une substance active désinfectante, permettant de répondre à ces objectifs, qu'une composition comprenant 5,4 à 11% en masse d'acide lactique à titre de substance active désinfectante, 1,955 à 4,45% en masse d'un agent tensio-actif non ionique à effet détergent et/ou mouillant appartenant au groupe comprenant au moins :
- alkyle polyglucosides ;
- PEG-75 lanoline ;
- polyoxyethylene-20-sorbitan monolaurate ;
- polymère d'oxyde de propylène et d'oxyde d'éthylène,
seul ou en mélange, un tampon de pH, et un agent régulateur de pH, pour ajuster le pH de la composition entre 2 et 4, de préférence entre 2,2 et 3 permettait d'y parvenir.

L'invention propose ainsi, de façon inédite, d'utiliser de l'acide lactique, issu par exemple du sucre de betterave, en association avec un agent tensio-actif non-ionique pour conférer à la composition une efficacité bactéricide convenable contre les bactéries les plus courantes, quelques minutes seulement après son application. Par ailleurs, les inventeurs ont constaté qu'une telle composition microbicide reste efficace vis-à-vis de la plupart des bactéries et de certaines levures entre deux traites, du fait de la synergie entre l'acide lactique et la ou les agents tensio-actifs non-ioniques.

L'agent régulateur de pH permet avantageusement d'ajuster la valeur du pH de la composition à une valeur inférieure, tout en restant proche, du pKa de l'acide lactique, à savoir 3,8.

Le tampon de pH peut par exemple être du citrate trisodique dihydraté, du lactate de sodium ou du phosphate tripotassique anhydre. Il permet de garantir une bonne stabilité de la composition dans le temps et de maintenir une concentration active en acide lactique suffisante.

Une composition selon l'invention peut comprendre un mélange de deux, trois ou quatre des agents tensio-actifs non-ioniques listés ci-dessus :
- alkyle polyglucosides ;
- PEG-75 lanoline ;
- polyoxyethylene-20-sorbitan monolaurate ;
- polymère d'oxyde de propylène et d'oxyde d'éthylène

Les alkyle polyglucosides, aussi connus sous l'acronyme APG, peuvent être du D-glucopyranose, oligomeric, C10-16 alkyl glucoside ou du D-glucopyranose, oligomeric, C8-10 alkyl glucoside ou un mélange de ces deux composés.

On notera que le polymère d'oxyde de propylène et d'oxyde d'éthylène est un agent mouillant qui limite avantageusement la formation de mousse et ne forme pas de goutte, ce qui permet d'obtenir un film homogène d'épaisseur régulière sur le trayon.

Dans un mode de réalisation particulièrement avantageux de l'invention, une telle composition désinfectante et filmogène comprend 0,655 à 2,05% en masse d'alkyle polyglucosides et 0,3 à 0,9% en masse de polymère d'oxyde de propylène et d'oxyde d'éthylène et 1 à 1,5% en masse de PEG-75 lanoline.

Dans un mode de réalisation particulier de l'invention, une composition désinfectante et filmogène telle que décrite ci-dessus comprend 0,1 à 0,675% d'oléate de glycéryle, qui assure notamment une fonction d'activateur lipidique de l'épiderme et d'émollient.

Préférentiellement, ledit composant filmogène est un acétate de vinyle polymérisé avec l'alcool vinylique, hydrolysé à au moins 98% et la proportion massique dudit composant filmogène dans ladite composition est comprise entre 3,5 et 5%.

L'acétate de vinyle polymérisé avec l'alcool vinylique, aussi connu sous l'acronyme PVA, forme, après séchage, un film souple et fin, qui autorise la respiration de la peau, tout en formant une barrière de protection contre les salissures.

Ce film polymérique non réticulé est suffisamment résistant pour permettre l'action prolongée de l'acide lactique entre deux traites, et suffisamment soluble dans l'eau et non collant pour être éliminé par un simple lavage lors de la préparation de la traite suivante.

Il est par ailleurs résistant aux frottements et aux conditions extérieures, telles que le froid, la pluie ou le vent.

Enfin il sèche rapidement.

Selon un mode de réalisation avantageux de l'invention, une composition telle que décrite ci-dessus comprend 0,8 à 1,5% en masse d'un agent tensio-actif anionique, tel que du lauryl éther sulfate de sodium, ou d'un mélange d'agents tensio-actifs anioniques.

Il convient de noter que l'association de lauryl éther sulfate de sodium associé à un alkyle polyglucoside est particulièrement efficace pour éliminer les levures de type *Candica albicans.*

Dans d'autres modes de réalisation de l'invention, il peut être prévu que ledit ou lesdits agents tensio-actifs anioniques soient du sodium lauroyl sarcosinate et/ou du alpha oléfine sulfonate de sodium.

Avantageusement, une telle composition comprend 5 à 10% en masse de glycérine.

La glycérine confère à la composition un effet émollient sur la peau du trayon et, le cas échéant, un effet plastifiant.

Dans un mode de réalisation particulier de l'invention, une composition désinfectante et filmogène telle que décrite ci-dessus comprend de la gomme de xanthane, destinée à épaissir ladite composition.

Il peut également être envisagé de mettre en oeuvre de l'hydroxyéthylcellulose pour épaissir la composition désinfectante et filmogène, dans un autre mode de réalisation de l'invention.

Cet agent épaississant permet d'ajuster la viscosité de la composition de façon à permettre une application aisée de la composition par trempage, sans dépôt excessif, mais également sans perdre de produit, par gouttage, lorsque la composition sèche et que le film se forme.

Dans un mode de réalisation particulier de l'invention, la proportion massique dudit agent régulateur de pH dans ladite composition est comprise entre 0,05 et 1,0%.

Le régulateur de pH peut par exemple être un mélange d'acide citrique, de citrate trisodique et d'une des bases suivantes : hydroxyde de sodium, iminodisuccinate de sodium ou N,Nbis(carboxyméthyl)-DL-alanine, triple sel de sodium.

Selon un aspect particulier de l'invention, la proportion massique dudit tampon de pH dans ladite composition est comprise entre 0,5 et 2%.

Dans un mode de réalisation particulier de l'invention, une composition désinfectante et filmogène telle que décrite ci-dessus comprend 0,01 à 0,5% en masse d'allantoïne.

### 5. Exemple de composition selon l'invention

A titre d'exemple, et de façon non limitative, la formulation d'une composition désinfectante et filmogène selon l'invention est détaillée dans le tableau suivant :

| **Composé** | **% massique** |
|---|---|
| Acide lactique | 5,600 % |
| D-glucopyranose, oligomeric, C8-10 alkyl glucoside | 0,700% |
| polymère d'oxyde de propylène et d'oxyde d'éthylène | 0,300 % |
| PEG-75 lanoline | 1,000 % |
| Citrate trisodique dihydraté | 0,650 % |
| Acide citrique | 0,200 % |
| Hydroxyde de sodium | 0,100 % |
| Acétate de vinyle polymérisé avec l'alcool vinylique, complètement hydrolysé | 4,396 % |
| lauryl éther sulfate de sodium | 0,810 % |
| Oléate de glycéryle | 0,450 % |
| Glycérine | 8,000 % |
| Allantoïne | 0,500 % |
| Méthanol | 0,13188% |
| Gomme de xanthane | 0,800 % |
| Parfum | 0,020 % |
| Colorant (orange) | 0,016 % |
| Eau déminéralisée | q.s.p. 100% |

Des tests suivant les normes EN 1656 et 1657 ont montré que cette composition est efficace en 5 minutes à 30°C dans des conditions de désinfection des trayons contre les souches d'*Escherichia Coli,* de *Streptococcus uberis* ou de *Staphylococcus aureus* et qu'elle inactive *Candida albicans.*

Un autre exemple de formulation d'une composition selon l'invention, désinfectante en 5 minutes, est donné dans le tableau ci-dessous:

| **Composé** | **% massique** |
|---|---|
| Acide lactique | 5,600 % |
| D-glucopyranose, oligomeric, C8-10 alkyl glucoside | 1,075 % |
| D-glucopyranose, oligomeric, C10-16 alkyl glucoside | 0,375% |
| polymère d'oxyde de propylène et d'oxyde d'éthylène | 0,600 % |
| PEG-75 lanoline | 1,500 % |
| Citrate trisodique dihydraté | 0,650 % |
| Acide citrique | 0,200 % |
| Hydroxyde de sodium | 0,100 % |
| Acétate de vinyle polymérisé avec l'alcool vinylique, complètement hydrolysé | 3,500 % |
| lauryl éther sulfate de sodium | 1,080% |
| Oléate de glycérile | 0,450 % |
| Glycérine | 8,000 % |
| Allantoïne | 0,500 % |
| Méthanol | 0,10502% |
| Gomme de xanthane | 0,400 % |
| Parfum | 0,020 % |
| Colorant (orange) | 0,016 % |
| Eau déminéralisée | q.s.p. 100% |

Cette formulation est moins concentrée en acide lactique que la précédente et plus faiblement filmogène, et elle assure une protection des trayons pendant au moins 1 heure.

Les inventeurs ont constaté qu'il était nécessaire pour obtenir une protection optimale, permettant de récupérer un film à la surface de la peau des trayons, que les proportions massiques minimales suivantes en polymère d'oxyde de propylène et d'oxyde d'éthylène, en lauryl éther sulfate de sodium et en tensio-actif non ionique soient respectées dans la formulation des compositions selon l'invention :
- pour 4% en masse d'acétate de vinyle polymérisé avec l'alcool vinylique, complètement hydrolysé, la proportion massique en polymère d'oxyde de propylène et d'oxyde d'éthylène doit être au minimum de 0,5%, celle en lauryl éther sulfate de sodium au minimum de 1,08% et celle en tensio-actif non ionique au minimum de 2,70%.
- pour 4,5% d'acétate de vinyle polymérisé avec l'alcool vinylique, complètement hydrolysé, la proportion massique en polymère d'oxyde de propylène et d'oxyde d'éthylène doit être au minimum de 0,3%, celle en lauryl éther sulfate de sodium au minimum de 0,81% et celle en tensio-actif non ionique au minimum de 2,00%.

Pour ces deux exemples, le pH doit par ailleurs être tamponné entre 2,20 et 3,00.

## Revendications

1. Composition désinfectante et filmogène destinée à être appliquée après une traite sur le trayon d'un bovin, d'un ovin ou d'un caprin comprenant en milieu aqueux un composant filmogène et au moins une substance active désinfectante, **caractérisée en ce qu'**elle comprend 5,4 à 11% en masse d'acide lactique à titre de substance active désinfectante et **en ce qu'**elle comprend en outre 1,955 à 4,45% en masse d'un agent tensio-actif non ionique à effet détergent et/ou mouillant mouillant appartenant au groupe comprenant au moins :
- alkyle polyglucosides ;
- PEG-75 lanoline ;
- polyoxyethylene-20-sorbitan monolaurate ;
- polymère d'oxyde de propylène et d'oxyde d'éthylène,
seul ou en mélange, un tampon de pH, et un agent régulateur de pH, pour ajuster le pH de la composition entre 2 et 4, de préférence entre 2,2 et 3.

2. Composition désinfectante et filmogène selon la revendication 1, **caractérisée en ce qu'**elle comprend 0,655 à 2,05% en masse d'alkyle polyglucosides et 0,3 à 0,9% en masse de polymère d'oxyde de propylène et d'oxyde d'éthylène et 1 à 1,5% en masse de PEG-75 lanoline.

3. Composition désinfectante et filmogène selon l'une quelconque des revendications 1 à 2, **caractérisée en ce qu'**elle comprend en outre 0,5 à 2% en masse d'oléate de glycéryle.

4. Composition désinfectante et filmogène selon l'une quelconque des revendications 1 à 3, **caractérisée en ce que** ledit composant filmogène est un acétate de vinyle polymérisé avec l'alcool vinylique, hydrolysé à au moins 98% et **en ce que** la proportion massique dudit composant filmogène dans ladite composition est comprise entre 3,5 et 5%.

5. Composition désinfectante et filmogène selon l'une quelconque des revendications 1 à 4, **caractérisée en ce qu'**elle comprend 0,8 à 1,5% en masse d'un agent tensio-actif anionique, tel que le lauryl éther sulfate de sodium, ou d'un mélange d'agents tensio-actifs anioniques.

6. Composition désinfectante filmogène selon l'une quelconque des revendications 1 à 5, **caractérisée en ce qu'**elle comprend 5 à 10% en masse de glycérine.

7. Composition désinfectante et filmogène selon l'une quelconque des revendications 1 à 6, **caractérisée en ce qu'**elle comprend de la gomme de xanthane, destinée à épaissir ladite composition.

8. Composition désinfectante et filmogène selon l'une quelconque des revendications 1 à 7, **caractérisée en ce que** la proportion massique dudit agent régulateur de pH dans ladite composition est comprise entre 0,05 et 1,0%.

9. Composition désinfectante et filmogène selon l'une quelconque des revendications 1 à 8, **caractérisée en ce que** la proportion massique dudit tampon de pH dans ladite composition est comprise entre 0,5 et 2%.

10. Composition désinfectante et filmogène selon l'une quelconque des revendications 1 à 9, **caractérisée en ce qu'**elle comprend 0,01 à 0,5% en masse d'allantoïne.
